# EUROPÄISCHE PATENTSCHRIFT

(11) **EP 3 635 043 B1**
(45) Veröffentlichungstag und Bekanntmachung des Hinweises auf die Patenterteilung: **15.02.2023**
(21) Anmeldenummer: 18729409.5
(22) Anmeldetag: 05.06.2018
(51) Int. Cl.: C08J 11/12, C10B 53/07, C08L 25/06

(54) **RECYCLINGVERFAHREN FÜR STYROL-HALTIGE KUNSTSTOFFABFÄLLE**
RECYCLING METHOD FOR STYRENE-CONTAINING PLASTIC WASTE
PROCÉDÉ DE RECYCLAGE POUR DÉCHETS PLASTIQUES CONTENANT DU STYRÈNE

(30) Priorität: 06.06.2017 US 201762515690 P
(43) Veröffentlichungstag der Anmeldung: 15.04.2020
(73) Patentinhaber: INEOS Styrolution Group GmbH, 60325 Frankfurt (DE)
(72) Erfinder: WILHELMUS, Bianca, 63456 Hanau (DE); NIESSNER, Norbert, 67159 Friedelsheim (DE); KERSCHBAUMER, Hannes, 65812 Bad Soden am Taunus (DE); ABBOUD, Mohammed, Riverside, Illinois 60546 (US)
(74) Vertreter: Jacobi, Markus Alexander
(86) Internationale Anmeldenummer: PCT/EP2018/064738
(87) Internationale Veröffentlichungsnummer: WO 2018/224482

(56) Entgegenhaltungen:
- EP-A1- 0 915 917
- DE-A1- 19 928 645
- JP-A- H0 789 900
- JP-A- H11 199 875
- JP-A- 2000 191 825
- JP-A- 2001 040 136
- JP-A- 2001 123 007
- US-A- 5 731 483
- MERTINKAT J ET AL: "Cracking catalysts used as fluidized bed material in the Hamburg pyrolysis process", JOURNAL OF ANALYTICAL AND APPLIED PYROLYSIS, vol. 49, no. 1, 1 January 1999 (1999-01-01), pages 87-95, XP085019786, ISSN: 0165-2370, DOI: 10.1016/S0165-2370(98)00103-X

## Beschreibung

Die Erfindung betrifft ein Verfahren zur wirtschaftlichen Nutzung von Styrol-haltigen Kunststoffabfällen als Rohstoff für hochwertige neue Kunststoff-Produkte im Rahmen eines rohstofflichen Recyclings.

Der Übergang von einer Linear- zu einer Kreislaufwirtschaft ist angesichts von Klimawandel, Umweltverschmutzung, Bevölkerungswachstum und Ressourcenabhängigkeit sowohl ökologisch als auch ökonomisch erforderlich. In den 1980er und 1990er Jahren wurden bereits intensive Anstrengungen zur Entwicklung von Verfahren für die rohstoffliche Wiederverwertung von Kunststoffabfällen unternommen, wobei es aufgrund ungelöster verfahrenstechnischer Problemstellungen und aus wirtschaftlichen Gründen bisher nicht zu großtechnischen Anwendungen kam. Bei der Verwertung von Kunststoffabfällen werden generell drei Arten unterschieden:
a) Thermische Verwertung
   Diese Verwertungsform ist die derzeit vorherrschende, da sie keine sortenähnlichen Stoffströme voraussetzt. Die Kunststoffabfälle werden ohne weitere Wiederverwertung verbrannt und daher lediglich die frei werdende Energie genutzt. Dieser Ansatz spart zwar die Nutzung äquivalenter Mengen Erdöl, erfüllt aber nicht die Anforderungen einer stofflichen, nachhaltigen Kreislaufwirtschaft und erzeugt vielfach umweltschädliche Stoffe. Die thermische Verwertung ist daher die ökologisch am wenigsten bevorzugte Verwertungsvariante.
b) Werkstoffliche Verwertung
   Bei der werkstofflichen Verwertung wird der Altkunststoff aufgeschmolzen und regranuliert, um danach erneut zu Produkten und Halbzeugen verarbeitet zu werden. Die Problematik besteht hier jedoch darin, dass eine Verschlechterung der Kunststoffqualität eintritt, wenn das granulierte Material nicht sortenrein ist ("Downcycling"). Das ist insbesondere bei "post-consumer"-Abfallströmen der Fall, die üblicherweise Mischungen aus Polymeren sind. Eine vollständig sortenreine Auftrennung ist sehr aufwändig und für mehrphasige Kunststoffe wie z.B. ABS kaum möglich. PET bildet eine Ausnahme mit einem existierenden Flaschen-Sammelsystem, bei dem der eingesammelte Abfall bereits relativ sortenrein ist. Bei Polystyrol überwiegt das innerbetriebliche Recycling für industrielle Abfälle. Bei jeder Form der werkstofflichen Verwertung werden die Polymere allerdings durch das erneute Aufschmelzen thermisch geschädigt, was das Molekulargewicht bzw. die Polymerkettenlänge reduziert und zu einer Verschlechterung der mechanischen Eigenschaften führt.
   Polymere, die über lange Zeit thermisch und/oder durch UV-Strahlung stark beansprucht wurden, was insbesondere auch auf Plastikmüll aus der Umwelt zutrifft, eignen sich schon aus diesem Grund für diese Form des Recyclings kaum.
c) Rohstoffliche Verwertung:
   Die rohstoffliche Verwertung von Kunststoffabfällen umfasst üblicherweise die Aufspaltung der Makromoleküle in Einzelteile bis hin zu Monomeren, die direkt oder nach einer Reinigung wieder zur Kunststoffsynthese eingesetzt werden können, ohne dass störende Verunreinigungen oder reduzierte Kettenlängen die Produktqualität beeinträchtigen. Der Kunststoff wird über das Monomer in den Kunststoff-Kreislauf zurückgeführt. Eine spezielle Form der rohstofflichen Verwertung ist der Einsatz von Kunststoffen als Reduktionsmittel in Hochöfen, wodurch auf den sonst üblichen Koks verzichtet werden kann. In Europa wird dieser Weg fast ausschließlich von Österreich beschritten, da diesem Verfahren entgegengehalten werden muss, dass es sich nicht um ein Recycling im Sinne der Wiederverwendung (geschlossener Kreislauf) handelt.

Eine rohstofflichen Verwertung von Altkunststoffen, die in der Erzeugung neuer Polymere münden kann, ist die thermisch induzierte Depolymerisation, die in der Literatur beschrieben ist (z.B. W. Kaminsky, Recycling of polymers by pyrolysis, in: Journal de Physique IV Colloque 03 (C7) (1993) C7-1543-C7-1552). Hierbei wird das Polymer thermisch in niedermolekulare Substanzen wie Monomere und Oligomere gespalten.

Die Pyrolyse (thermische Zersetzung) von Kunststoffen ist bekannt. So beschreibt DE 2310463 ein Verfahren, bei dem die Depolymerisation in einem konventionellen Doppelschneckenextruder durchgeführt wird. Durch einen hohen Scherenergieeintrag wird das Material dissipativ erwärmt. Dadurch wird die Zersetzungstemperatur überschritten und das Material zersetzt. Die flüchtigen Monomere werden über entsprechende Vorrichtungen aufgefangen, während der schmelzeförmige Rückstand aus dem Extruder herausextrudiert wird.

Eine weitere Möglichkeit ist die Zersetzung im Wirbelschichtreaktor (W. Kaminsky, J. Menzel, H. Sinn, Recycling of plastics, in Conservation & Recycling, Vol.1 (1976) 91-110). Ein ähnliches Verfahren ist auch in EP 0649827 beschrieben. Die Zersetzung von Kunststoffen kann auch im Mikrowellenreaktor geschehen, wie in WO 2015/024102 beschrieben. Dieses Verfahren erzeugt aus gemischtem Haushaltsabfall ein Gas oder ein Öl, welches als Treibstoff oder für weitere chemische Syntheseschritte verwendet werden kann. WO 2011/079894 beschreibt die Depolymerisation von unsortierten Polymermischungen in einem Reaktor unter Zufuhr von thermischer Energie und mit Hilfe eines Katalysators.

Die Verwendung von Katalysatoren hat im Allgemeinen bei der Pyrolyse von Kunststoffabfall, insbesondere Styrol-haltigem Kunststoffabfall, den Nachteil, dass sich aufgrund der aromatischen Molekularstruktur und des großen C/H-Verhältnis des Styrols auch relativ viel Kohlenstoff bildet, der sich auf der Katalysatoroberfläche ablagert und diesen schnell unbrauchbar macht.

Die thermische Zersetzung führt nicht bei allen Polymertypen zu direkten Ausgangsstoffen für eine erneute Polymerisation. So ergibt die thermische Zersetzung von Polyethylen, Polypropylen und Polyester nur unspezifische Produkte wie Wachse, Leicht-öl und Gase, welche sich nicht für eine erneute Polymerisation eignen, sondern wirtschaftlich nur als Brennstoff verwendet werden können. Polystyrol (PS) dagegen gehört zu den Polymeren, die sich bei höheren Temperaturen wieder in ihre Monomere aufspalten lassen und so für eine erneute Polymerisation geeignet sind. Polystyrol, das in vielen Bereichen des täglichen Lebens und auch in Einwegverpackungen zum Einsatz kommt, eignet sich daher für die hier angestrebte rohstoffliche Verwertung in besonderem Maße.

Wird Polystyrol stark erhitzt, so bilden sich Zersetzungsprodukte. Dabei entstehen vor allem Styrol-Monomere, bei unvollständiger Zersetzung auch Oligomere in der Form von z.B. Dimeren, Trimeren und Tetrameren, bei zu starker Zersetzung auch Benzol, Toluol, Ethylbenzol und α-Methylstyrol. Die Anteile der einzelnen Bestandteile variieren und hängen stark von den experimentellen Bedingungen, insbesondere von der Temperatur und dem Molekulargewicht des Polystyrols ab (C. Bouster, Study of the pyrolysis of polystyrenes: 1. Kinetics of thermal decomposition, in: Journal of Analytical and Applied Pyrolysis, 1 (1980) 297-313; C. Bouster, Evolution of the product yield with temperature and molecular weight in the pyrolysis of polystyrene, in: Journal of Analytical and Applied Pyrolysis 15 (1989) 249-259).

Für die Re-Polymerisierung können jedoch nur die Styrol-Monomere verwendet werden. Styrol-Oligomere sind beim erneuten Polymerisieren störend, da sie selbst in geringen Mengen bereits wesentliche Eigenschaften des Polymers beeinflussen, und sind deswegen nicht erwünscht. Das Problem ist, dass die bei der Zersetzung des Polymers gebildeten Styrol-Monomere in einer 4+2-Diels-Alder-Cycloadditionsreaktion bereits wieder zu Oligomeren reagieren, wenn sie nicht schnell genug abgekühlt werden (siehe dazu Kirchner, K. und Riederle, K., "Thermal polymerization of styrene - the formation of oligomers and intermediates, 1. Discontinuous polymerization up to high conversions", in: Die Angewandte Makromolekulare Chemie 111 (1983) 1-16).

Es existieren zwei mögliche Quellen für Styrol-Oligomere: zum einen die unvollständige Zersetzung des Ausgangspolymers, zum anderen die Neubildung aus Styrol-Monomeren nach der Pyrolyse.

KR 2004-0088685 beschreibt eine Methode zur Rückgewinnung von Styrol aus Polystyrol-haltigen Abfällen durch Pyrolyse bei 200 bis 1000 °C, es wird jedoch nicht auf das Problem der Oligomerenbildung eingegangen.

In US 6,018,085 erläutert eine Methode zur Gewinnung von Styrol aus Polystyrol-haltigen Materialien, die mit tierischen oder pflanzlichen Fetten verunreinigt sind, durch thermische Depolymerisierung einer Lösung der Materialien in einem Lösungsmittel bei 300-350 °C, jedoch ohne Hinweis auf Oligomeren-Bildung oder Wege, diese zu verhindern.

US 9,650,313 offenbart die thermische Depolymerisation von Polystyrol, welches aus einem Polystyrol-haltigen Abfall herausgelöst wurde, bei 330-800 °C. Auch hier wird das Problem der Oligomerenbildung nicht behandelt.

US 3,901,951 beschreibt die Pyrolyse von Polystyrol, liefert aber keine Lösung zur Minimierung der Oligomerenbildung oder zur Nutzung von Oligomeren.

DE 199 28 645 und JP 11 199875 beschreiben weitere Verfahren zur Pyrolyse von Polystyrol bei niedrigem Druck.

EP 0 915 917 beschreibt die thermische Depolymerisation von Polymeren wie Poly(methylmethacrylat), um daraus Monomere zu erhalten.

In dem Artikel "Cracking catalysts used as fluidized bed material in the Hamburg pyrolysis process" (J. Mertinkat et al., Journal of Analytical and Applied Pyrolysis, 1999, 49, Seiten 87-95) wird die thermische Depolymerisation von Polystyrol und Polyethylen im Wirbelschichtreaktor in Anwesenheit von Katalysatoren beschrieben.

Die Bildung der Oligomere hängt im Wesentlichen von der Temperatur und der Verweilzeit im Reaktor ab. Je höher die Temperatur und je länger die Verweilzeit sind, desto mehr unerwünschte Oligomere entstehen. Die Temperatur und die Verweilzeit im Reaktor können jedoch nicht beliebig verringert werden, da zur Zersetzung des Kunststoffabfalls eine Mindesttemperatur und Mindestverweilzeit notwendig sind. Einem Fachmann ist derzeit kein Verfahren bekannt, wie die Oligomeren-Bildung vollständig verhindert werden kann.

Auch ist gerade wegen der Vielzahl der möglichen Verfahren dem Fachmann nicht klar, unter welchen Bedingungen die Ausbeute an Styrol im industriellen Maßstab maximiert werden kann. Tabelle 1 listet einige in der Literatur beschriebene Verfahren und die dort berichteten Ausbeuten auf. Für den industriellen Maßstab gelten Ausbeuten von >95% als ausreichend.

**Tabelle 1: Exemplarische Auflistung einiger Pyrolyseverfahren zum Recycling von Polystyrol und ihre Ausbeuten**

| Literatu rstel le | Reaktionsbedingungen | Ausbeute an Styrol-Monomer | Gesamtausbeute an Styrol-Monomer, -dimer und -trimer |
|---|---|---|---|
| C. Bouster, Evolution of the product yield with temperature and molecularweight in the pyrolysis of polystyrene, Journal of Analytical and Applied Pyrolysis 15 (1989) 249-259 | Flashpyrolyse bei 500 bis 975 °C | 66,8% bis 78,7%, abhängig von der Reaktionstemperatur | 70,3% bis 86,4%, abhängig von der Reaktionstemperatur |
| G. Audisio, Molecular weight and pyrolysis products distribution of polymers. Polystyrene, in: Journal of Analytical and Applied Pyrolysis, 24(1) (1992) 61-74 | Pyrolyse bei 600 °C | 79,5% bis 90,7%, abhängig von der Molmasse des verwendeten Polystyrols | 79,5% bis 90,7%, abhängig von der Molmasse des verwendeten Polystyrols |
| M. Bartoli, Depolymerization of polystyrene at reduced pressure through a microwave assisted pyrolysis, in: Journal of Analytical and Applied Pyrolysis, 113 (2015) 281-287 | Mikrowellen unterstützte Pyrolyse | 36,7% bis 50,1%, abhängig von den Reaktionsbedingungen | 38,5% bis 59,5%, abhängig von den Reaktionsbedingungen |
| Y. S. Kim, Pyrolysis of polystyrene in a batchtype stirred vessel. Korean Journal of Chemical Engineering, 16(2) (1999) 161-165 | Pyrolyse bei 370 bis 400 °C | 65,4% bis 71,6%, abhängig von der Reaktionstemperatur | 87,5% bis 94,6%, abhängig von der Reaktionstemperatur |
| S. Ide, Controlled Degradation of Polystyrene, in: J Appl Polym Sei, 29 (1984), 2561-2571. | Pyrolyse bei 350 bis 500 °C | 35,4 bis 64,5%, abhängig von der Reaktionstemperatur | 72,6% bis 77,8%, abhängig von der Reaktionstemperatur |
| R. Lin, Acid-Catalyzed Cracking of Polystyrene, J Appl Polym Sei, 63 (1997) 1287-1298. | Pyrolyse bei 400 °C | 41% bis 68%, abhängig von der Molmasse des verwendeten Polystyrols | 87% bis 90%, abhängig von der Molmasse des verwendeten Polystyrols |

Es besteht demnach ein Bedarf an Verfahren der thermischen Zersetzung von Kunststoffabfall, bei denen eine maximale Menge an Monomer sowie weiteren direkt petrochemisch nutzbaren Rohstoffen entsteht und bei denen die Styrol-Oligomere als Nebenprodukt möglichst vollständig abgetrennt und einer weiteren petrochemischen Nutzung zugeführt werden. Direkt petrochemisch nutzbare Rohstoffe sind beispielsweise Styrol, Benzol, Ethen oder andere Naphtha-Bestandteile. Unter Styrol-Oligomeren werden Styrol-Dimere, Styrol-Trimere und weitere Verbindungen mehrerer Styrol-Monomere miteinander verstanden.

Eine Aufgabe der Erfindung ist es, ein rohstoffliches Recyclingverfahren für Styrol-haltige Kunststoffabfälle bereitzustellen, dessen Rohstoffe aus unterschiedlichen Kunststoffen bestehen können und das eine hohe Menge an Styrol-Monomer neben weiteren petrochemisch nutzbaren Rohstoffen erzeugt.

Die Aufgabe wird gelöst durch ein Verfahren zur pyrolytischen Depolymerisation eines Styrol-haltigen Kunststoffabfalls (K), enthaltend oder bestehend aus den Komponenten A, B1, B2, B3 und C:
A: 10 bis 100 Gew.-%, bezogen auf die Summe der Komponenten A, B1, B2 und B3, mindestens eines Styrol-haltigen Polymers, enthaltend mindestens 40% Styrol, bevorzugt mindestens 50 Gew.-% Styrol, besonders bevorzugt mindestens 80 Gew.-% Styrol, insbesondere mindestens 85 Gew.-% Styrol, jeweils bezogen auf die Komponente A, und bis zu 60 Gew.-%, bezogen auf die Komponente A, Kautschuk und/oder weitere Comonomere, die den Pyrolyseprozess nicht unterbrechen, insbesondere Acrylnitril, Vinychlorid, Methylmethacrylat und/oder alpha-Methylstyrol, bevorzugt weniger als 20 Gew.-% Acrylnitril, Vinychlorid, Methylmethacrylat und/oder alpha-Methylstyrol, besonders bevorzugt weniger als 10 Gew.-% Acrylnitril, Vinychlorid, Methylmethacrylat und/oder alpha-Methylstyrol;
B1: 0 bis 60 Gew.-%, bezogen auf die Summe aus A, B1, B2 und B3, Polyolefin oder Polyolefin-Mischungen, wie Polyethylen oder Polypropylen;
B2: 0 bis 60 Gew.-%, bezogen auf die Summe aus A, B1, B2 und B3, weitere von A und B1 verschiedene Polymere, beispielsweise Polycarbonate, Polyester, Polyamide und/oder Polyvinylchlorid;
B3: 0 bis 20 Gew.-%, bezogen auf die Summe aus A, B1, B2 und B3, übliche Kunststoff-Additive und -Hilfsstoffe;
C: 0 bis 50 Gew.-%, bezogen auf die Summe aus A, B1, B2 und B3, sonstige Fremdstoffe, Schmutz und Feuchtigkeit,
umfassend die folgenden Schritte (bzw. bestehend aus den Schritten):
i) Zersetzen des Styrol-haltigen Kunststoffabfalls (K) in einem geeigneten Reaktor ohne Katalysator durch Einbringen von thermischer Energie und gegebenenfalls Scherenergie, wobei der zu zersetzende Kunststoffabfall (K) einer Pyrolysezone des Reaktors zugeführt und dort bei einer Temperatur von 250 °C bis 500 °C (gemessene (mittlere) Materialtemperatur des Reaktionsgutes an der inneren Oberfläche einer Reaktorwand des Reaktors (während der Reaktionslaufzeit) pyrolysiert wird, wobei die Verweilzeit des zu pyrolysierenden Kunststoffabfalls (K) in der Pyrolysezone 1 bis 45 Minuten beträgt, und wobei die Styrol-Komponente (A) des Styrol-haltigen Kunststoffabfalls (K) zumindest teilweise zu Styrol-Monomeren und Styrol-Oligomeren zersetzt wird;
ii) Abführen und Auffangen der in Schritt i) entstehenden Gase und Kondensation der niedermolekularen Produkte, enthaltend die gebildeten Styrol-Monomere, in einem geeigneten Abscheider.
iii) Auftrennung der kondensierten niedermolekularen Bestandteile des vorherigen Schrittes, enthaltend die gebildeten Styrol-Monomere, mittels fraktionierender Destillation und
iv) Optional Zuführen der in Schritt i) gebildeten sowie gegebenenfalls bereits vorhandenen Styrol-Oligomere zu einem Steamcracker.

Das erfindungsgemäße Verfahren liefert eine hohe Ausbeute an Styrol-Monomeren und ermöglicht eine weitere petrochemische Nutzung des Styrol-Oligomeranteils.

### Styrol-haltiger Kunststoffabfall (K)

Als Ausgangsmaterial eignen sich für das erfindungsgemäße Verfahren Styrol-haltige Kunststoffabfälle (K), enthaltend die (und vorzugsweise bestehend aus den) Komponenten A, B1, B2, B3 und C:
A: Styrol-haltiges Polymer mit mindestens 40 Gew-% Styrol, bevorzugt mindestens 50 Gew.-% Styrol, besonders bevorzugt mindestens 80 Gew.-% Styrol, insbesondere mindestens 85 Gew.-% Styrol.

Weitere Bestandteile der Komponente A sind gegebenenfalls bis zu 60 Gew.-%, bevorzugt 50 Gew.-%, bezogen auf die Komponente A, Kautschuk und/oder weitere Comonomere, die den Pyrolyseprozess nicht unterbrechen, insbesondere Acrylnitril, Vinylchlorid, Methylmethacrylat oder alpha-Methylstyrol. Bevorzugt sind weniger als 20 Gew.-% Acrylnitril, Vinylchlorid, Methylmethacrylat oder alpha-Methylstyrol, besonders bevorzugt weniger als 10 Gew.-% Acrylnitril, Vinylchlorid, Methylmethacrylat oder alpha-Methylstyrol.

Die Menge an Komponente A in dem Styrol-haltigen Kunststoffabfall (K) beträgt 10 bis 100 Gew.-%, bevorzugt mindestens 50 Gew.-%, jeweils bezogen auf die Summe der Komponenten A, B1, B2 und B3.

B1: 0 bis 60 Gew.-%, bezogen auf die Summe aus A, B1, B2, und B3, Polyolefine und Polyolefin-Mischungen, wie Polyethylen, Polypropylen.

B2: 0 bis 60 Gew.-%, bezogen auf die Summe aus A, B1, B2, und B3, weitere von A und B1 verschiedene Polymere, beispielsweise Polycarbonate, Polyester, Polyamide und/oder Polyvinylchlorid.

B3: 0 bis 20 Gew.-%, bezogen auf die Summe aus A, B1, B2, B3, übliche Kunststoff-additive und -Hilfsstoffe. Beispielhaft kann ein Additiv oder ein Hilfsstoff ausgewählt sein aus der Gruppe bestehend aus Antioxidantien, UV-Stabilisatoren, PeroxidZerstörern, Antistatika, Gleitmitteln, Entformungsmitteln, Flammschutzmitteln, Füll- oder Verstärkerstoffen (Glasfasern, Kohlefasern, etc.), Farbmitteln und Kombinationen aus zwei oder mehreren davon.

C: 0 bis 50 Gew.-%, bezogen auf die Summe aus A, B1, B2, B3, sonstige Fremdstoffe, Schmutz und Feuchtigkeit. Gegebenenfalls ist das Ausgangsmaterial wie oben beschrieben durch Waschprozesse soweit vorzubehandeln, dass nicht mehr als 50 Gew.-%, bevorzugt nicht mehr als 30 Gew.-% und besonders bevorzugt nicht mehr als 20 Gew.-% sonstige Fremdstoffe, Schmutz und Feuchtigkeit enthalten sind, bezogen auf die Summe aus A, B1, B2 und B3.

Der erfindungsgemäß eingesetzte Styrol-haltige Kunststoffabfall (K) wird insbesondere bei einem höheren Anteil von C) gegebenenfalls in geeigneter Weise vorbehandelt, um weitere Stoffe oder Zusammensetzungen zu entfernen, beispielsweise anhaftende Verunreinigungen wie etwa Lebensmittelreste oder Schmutz, Feuchtigkeit und Fremdstoffe wie Metalle oder andere Stoffe und Verbundmaterialien.

Dies erfolgt vorteilhaft in einer Vorbehandlung, die einen oder mehrere der folgenden Schritte umfassen kann, wobei die Reihenfolge der Schritte nicht festgelegt ist und Schritte auch mehrfach wiederholt werden können: manuelle Störstoffsortierung, Wäsche, Zerkleinerung, automatische Sortierung in geeigneten Anlagen. Gegebenenfalls können auch Styrol-haltige Kunststoffabfälle, die außerhalb der Spezifikation der Kunststoffabfälle (K) liegen, durch ein solches Verfahren in erfindungsgemäß eingesetzte Materialien (K) umgewandelt werden, um so einen Styrol-haltigen Kunststoffabfall mit den Spezifikationen von (K) zu erhalten.

Bevorzugt ist daher auch eine Ausführungsform des erfindungsgemäßen Verfahrens, bei der ein Styrol-haltiger Kunststoffabfall (K) in einem Schritt o) einer Vorbehandlung unterworfen wird, die einen oder mehrere der folgenden Schritte umfasst, wobei die Reihenfolge der Schritte nicht festgelegt ist und Schritte auch mehrfach wiederholt werden können: manuelle Störstoffsortierung, Wäsche, Zerkleinerung, automatische Sortierung in geeigneten Anlagen, um so den erfindungsgemäßen Styrol-haltigen Kunststoffabfall (K) zu erhalten.

Bevorzugt enthält die Komponente A:
Styrol-haltiges Polymer A mit A1: mindestens 40 Gew-% Styrol, bevorzugt mindestens 50 Gew.-% Styrol, besonders bevorzugt mindestens 80 Gew.-% Styrol, insbesondere mindestens 85 Gew.-% Styrol, und A2: weiteren Conomomeren wie beispielsweise Butadien, (Meth)acrylat, Acrylnitril, alpha-Methylstyrol, Phenylmaleinimid.

Komponente A ist weiterhin bevorzugt ausgewählt aus der Klasse der nicht schlagfesten oder schlagfest modifizierten Polystyrole, aus den Vinylaromat-Copolymeren ausgewählt aus der Gruppe bestehend aus: Styrol-Acrylnitril-Copolymeren, α-Methylstyrol-Acrylnitril-Copolymeren, Styrol-Maleinsäureanhydrid-Copolymeren, Styrol-Phenylmaleinimid-Copolymeren, Styrol-Methylmethacrylat-Copolymeren, Styrol-Acrylnitril-Maleinsäureanhydrid-Copolymeren, Styrol-Acrylnitril-Phenylmaleinimid-Copolymeren, α-Methylstyrol-Acrylnitril-Methylmethacrylat-Copolymeren, α-Methylstyrol-Acrylnitril-t-Butylmethacrylat-Copolymeren und Styrol-Acrylnitril-t-Butylmethacrylat-Copolymeren.

Schlagzähmodifikatoren A3 können optional ebenfalls enthalten sein.

Diese bestehen beispielsweise aus A31: 20-90 Gew.-% einer Pfropfgrundlage eines oder mehrerer Monomere, bestehend aus:
A311: 70 bis 100 Gew.-% mindestens eines konjugierten Diens und/oder mindestens eines Acrylats;
A312: 0 bis 30 Gew.-% mindestens eines weiteren Comonomers ausgewählt aus: Styrol, α-Methylstyrol, Acrylnitril, Methacrylnitril, MMA, MSA und N-Phenylmaleinimid (N-PMI);
A313: 0 bis 10 Gew.-% mindestens eines oder mehrerer polyfunktioneller, vernetzender Monomere, die, falls Komponente A211 Acrylat ist, in Mengen von mindestens 0.1 Gew % vorhanden ist,
A32: 10 bis 80 Gew.-% einer Pfropfauflage eines Monomers oder mehrerer Monomere, bestehend aus:
   A321: 65 bis 100 Gew.-%, bevorzugt 70 bis 100 Gew.-%, besonders bevorzugt 75 bis 95 Gew.-% mindestens eines vinylaromatischen Monomers, vorzugsweise Styrol und/oder α-Methylstyrol, insbesondere Styrol;
   A322: 5 bis 35 Gew.-%, bevorzugt 10 bis 30 Gew.-%, besonders bevorzugt 15 bis 25 Gew.-% Acrylnitril und/oder Methacrylnitril, vorzugsweise Acrylnitril,
   A323: 0 bis 30 Gew.-%, bevorzugt 0 bis 20 Gew.-%, besonders bevorzugt 0 bis 15 Gew.-% mindestens eines weiteren monoethylenisch ungesättigten Monomers ausgewählt aus: MMA, MSA und N-PMI;
wobei die Summe aus A31 und A32 100 Gew.-% ergibt, bezogen auf A3, und wobei der Anteil an Comonomeren A2 am Gesamt-Anteil der Komponente A nicht höher als 30 Gew,-%, bevorzugt nicht höher als 15 Gew.-%, besonders bevorzugt nicht höher als 8 Gew.-% und insbesondere nicht höher als 5 Gew.-% ist.

Bevorzugt als Polymer A sind Polystyrol (unmodifiziertes Standard-Polystyrol und/oder schlagfest modifiziertes Polystyrol), Styrol-Acrylnitril-Copolymere (SAN), Styrol-Methylmethacrylat-Copolymere (SMMA) und/oder Styrol-Maleinsäureanhydrid-Copolymere (SMSA). Besonders bevorzugt sind Styrol-Acrylnitril-Copolymere.

Als erfindungsgemäße Polymere A eingesetzte SAN-Copolymere und α-Methylstyrol-Acrylnitril-Copolymere (AMSAN) enthalten im Allgemeinen 18 bis 35 Gew.-%, bevorzugt 20 bis 32 Gew.-%, besonders bevorzugt 22 bis 30 Gew.-% Acrylnitril (AN), und 82 bis 65 Gew.-%, bevorzugt 80 bis 68 Gew.-%, besonders bevorzugt 78 bis 70 Gew.-% Styrol (S) bzw. α-Methylstyrol (AMS), wobei die Summe aus Styrol bzw. α-Methylstyrol und Acrylnitril 100 Gew.-% ergibt. Die SAN und AMSAN-Copolymere weisen im Allgemeinen eine mittlere Molmasse Mw von 50.000 bis 500.000 g/mol, bevorzugt 100.000 bis 350.000 g/mol, besonders bevorzugt 100.000 bis 300.000 g/mol, und ganz besonders bevorzugt 150.000 bis 250.000 g/mol auf.

Erfindungsgemäß als Polymer A eingesetzte SMMA-Copolymere enthalten im Allgemeinen 18 bis 50 Gew.-%, bevorzugt 20 bis 30 Gew.-% Methylmethacrylat (MMA), und 50 bis 82 Gew.-%, bevorzugt 80 bis 70 Gew.-% Styrol, wobei die Summe aus Styrol und MMA 100 Gew.-% ergibt.

Erfindungsgemäß als Polymer A eingesetzte SMSA-Copolymere enthalten im Allgemeinen 10 bis 40 Gew.-%, bevorzugt 20 bis 30 Gew.-% Maleinsäureanhydrid (MSA), und 60 bis 90 Gew.-%, bevorzugt 80 bis 70 Gew.-% Styrol, wobei die Summe aus Styrol und MSA 100 Gew.-% ergibt.

Als konjugierte Diene kommen Diene mit 4 bis 8 Kohlenstoffatomen wie Butadien, Isopren, Piperylen und Chloropren oder deren Mischungen in Betracht. Bevorzugt verwendet man Butadien oder Isopren oder deren Mischungen, ganz besonders bevorzugt Butadien.

Dien-Kautschuke sind beispielsweise Homopolymere der vorgenannten konjugierten Diene, Copolymere solcher Diene untereinander, Copolymere solcher Diene mit Acrylaten, insbesondere n-Butylacrylat, und Copolymere solcher Diene mit den Comonomeren ausgewählt aus Styrol, α-Methylstyrol, Acrylnitril, Methacrylnitril, Methylmethacrylat (MMA), Maleinsäureanhydrid (MSA) und N-Phenylmaleinimid (N-PMI). Die Dien-Kautschuke können auch vernetzend wirkende, polyfunktionelle Monomere wie vorstehend für die Acrylatkautschuke genannt enthalten.

### Komponente B1

Als Komponente B1 kommen beispielsweise Polyolefine wie PE-LD (Low Density Polyethylen), PE-LLD (Linear Low Density Polyethylen), PE-HD (High Density Polyethylen), Metallocen-Polyethylene, Ethylen-Copolymere wie Poly(ethylen-co-vinylacetat), Ethylen-Buten, Ethylen-Hexen, Ethylen-Octen-Copolymere, sowie Cycloolefincopolymere einzeln oder als Mischung in Frage. Darüber hinaus kommen als Komponente B1 beispielsweise Polypropylen, wie Homo- oder Copolymere des Propylens, Metallocenkatalysierte Polypropylene sowie Copolymere des Propylens mit anderen, dem Fachmann bekannten Comonomeren in Betracht.

Die Komponente B ist in dem erfindungsgemäß eingesetzten Styrol-haltigen Kunststoffabfall (K) in einem Anteil von 0 bis 60 Gew.-%, bezogen auf die Summe der Komponenten A, B1, B2 und B3, in einer bevorzugten Ausführungsform von 0,1 bis 50 Gew.-%, bezogen auf die Summe der Komponenten A, B1, B2 und B3, enthalten.

### Komponente B2

Der erfindungsgemäß eingesetzte Styrol-haltige Kunststoffabfall (K) kann zusätzlich 0 bis 60 Gew.-%, bezogen auf die Summe aus A, B1, B2, B3, mindestens ein weiteres, von den Polymeren A und B1 verschiedenes Polymer B2 enthalten, beispielsweise ausgewählt aus Polycarbonaten, Polyamiden, Poly(meth)acrylaten, Polyvinylchloriden, Polyester, halogenierten Polymeren, Vinylaromat-Dien-Copolymeren (SBC), Polyethern, Polysulfonen, Polyethersulfonen, Polyimidazolen und verwandten Polymeren.

### Komponente B3

Als Komponente B3 können 0 bis 20 Gew.-%, bezogen auf die Summe aus A, B1, B2, B3, übliche Kunststoffadditive und -Hilfsstoffe enthalten sein. Beispielshaft kann ein Additiv oder ein Hilfsstoff ausgewählt sein aus der Gruppe bestehend aus Antioxidantien, UV-Stabilisatoren, Peroxidzerstörern, Antistatika, Gleitmitteln, Entformungsmitteln, Flammschutzmitteln, Füll- oder Verstärkerstoffen (Glasfasern, Kohlefasern, etc.), Farbmitteln und Kombinationen aus zwei oder mehr daraus. Als Beispiele für Oxidationsverzögerer und Wärmestabilisatoren seien Halogenide von Metallen der Gruppe I des periodischen Systems, z.B. Natrium-, Kalium- und/oder Lithiumhalogenide, ggf. in Verbindung mit Kupfer-(I)-Halogeniden, z.B. Chloriden, Bromiden, Jodiden, sterisch gehinderte Phenole, Hydrochinone, verschiedene substituierte Vertreter dieser Gruppen und deren Mischungen in Konzentrationen bis zu 1 Gew.-%, bezogen auf das Gewicht der Summe aus A, B1, B2 und B3 genannt.

Als UV-Stabilisatoren, die im Allgemeinen in Mengen bis zu 2 Gew.-%, bezogen auf die Summen aus A, B1, B2 und B3 enthalten sind, seien verschiedene substituierte Resorcine, Salicylate, Benzotriazole und Benzophenone genannt.

Weiterhin können organische Farbstoffe wie Nigrosin, Pigmente wie Titandioxid, Phthalocyanine, Ultramarinblau und Ruß als Farbstoffe im erfindungsgemäß eingesetzten Styrol-haltigen Kunststoffabfall (K) enthalten sein, sowie faser- und pulverförmige Füllstoffe und Verstärkungsmittel. Beispiele für letztere sind Kohlenstoffasern, Glasfasern, amorphe Kieselsäure, Calciumsilicat (Wollastonit), Aluminiumsilicat, Magnesiumcarbonat, Kaolin, Kreide, gepulverter Quarz, Glimmer und Feldspat. Der Anteil derartiger Füll- und Farbstoffe beträgt im Allgemeinen bis zu 50 Gew.-%, bevorzugt bis zu 35 Gew.-%.

Als Keimbildungsmittel können z.B. Talkum, Calciumfluorid, Natriumphenylphosphinat, Aluminiumoxid, Siliciumdioxid und Nylon 22 enthalten sein.

Gleit- und Entformungsmittel, welche in der Regel in Mengen bis zu 1 Gew.-% eingesetzt werden können, sind z.B. langkettige Fettsäuren wie Stearinsäure oder Behensäure, deren Salze (z.B. Ca- oder Zn-Stearat) oder Ester (z.B. Stearylstearat oder Pentaerythrittetrastearat) sowie Amidderivate (z.B. Ethylenbisstearylamid).

Weiterhin können Antiblockmittel auf mineralischer Basis in Mengen bis zu 0,1 Gew.-% enthalten sein. Als Beispiele seien amorphe oder kristalline Kieselsäure, Calciumcarbonat oder Aluminiumsilikat genannt.

Als Verarbeitungshilfsmittel kann beispielsweise Mineralöl, vorzugsweise medizinisches Weißöl, in Mengen bis zu 5 Gew.-%, vorzugsweise bis zu 2 Gew.-% enthalten sein.

Als Beispiele für Weichmacher seien Phthalsäuredioctylester, Phthalsäuredibenzylester, Phthalsäurebutylbenzylester, Kohlenwasserstofföle, N-(n-Butyl)benzolsulfonamid und o- und p-Tolylethylsulfonamid genannt.

Weiterhin können alle für die jeweiligen Thermoplaste bekannten Flammschutzmittel enthalten sein, insbesondere solche auf Basis von Phosphorverbindungen oder roter Phosphor selbst.

### Schritt i

In Schritt (i) des erfindungsgemäßen Verfahrens erfolgt Zersetzen des Styrol-haltigen Kunststoffabfalls (K) in einem geeigneten Pyrolysereaktor ohne Katalysator durch Einbringen von thermischer Energie und optional Scherenergie. Dazu wird das zu zersetzende Material einer Pyrolysezone des Reaktors zugeführt und dort bei einer Temperatur von 250 °C bis 500 °C (gemessene (mittlere) Materialtemperatur des Reaktionsgutes an der inneren Oberfläche der Reaktorwand des Pyrolysereaktors während der Reaktionslaufzeit) pyrolysiert, wobei die Verweilzeit des zu pyrolysierenden Materials in der Pyrolysezone 1 bis 45 Minuten beträgt. Besonders bevorzugt sind Pyrolysetemperaturen von 280 °C bis 470 °C, besonders bevorzugt sind Pyrolysetemperaturen von 300 °C bis 450 °C und Verweilzeiten von 2 bis 30 Minuten.

Als Pyrolysereaktoren eignen sich beispielsweise die oben erwähnten Doppelschneckenextruder, Wirbelschichtreaktoren und Mikrowellenreaktoren.

Das Einbringen thermischer Energie kann beispielsweise durch Erwärmen oder Mikrowellenbestrahlung erfolgen.

Der erfindungsgemäß eingesetzte Pyrolysereaktor enthält keinen Katalysator.

In einer weiteren bevorzugten Ausführungsform wird dem Styrol-haltigen Kunststoffabfall (K) neben thermischer Energie zusätzlich Scherenergie zugeführt.

### Schritte ii und iii

In Schritt ii) erfolgt das Abführen und Auffangen der in Schritt i) entstehenden Gase und Kondensation der niedermolekularen Produkte, enthaltend die in Schritt i) gebildeten Styrol-Monomere, in einem geeigneten Abscheider. Schritt iii) beinhaltet die Auftrennung der aufgefangenen niedermolekularen Bestandteile des vorherigen Schrittes mittels fraktionierender Destillation. Die abgetrennten Styrol-Monomere stehen dann für eine erneute Polymerisation zur Verfügung. Geeignete Vorrichtungen für die Schritte ii) und iii) sind bekannt und dem Fachmann geläufig.

### Schritt iv)

Der (optionale) Schritt iv) des erfindungsgemäßen Verfahrens besteht schließlich im Zuführen der in Schritt i) gebildeten sowie gegebenenfalls bereits vorhandenen Styrol-Oligomere zu einem Steamcracker, in dem die Oligomere weiter aufgespalten werden, so dass auch aus diesem Material noch Ausgangsstoffe für Kunststoffe gewonnen werden können, wie etwa Ethen, Propen oder Benzol.

Das Steamcracken ist ein thermisches Zersetzungsverfahren, welches in Gegenwart von Wasserdampf durchgeführt wird. Es stellt den industriell wichtigsten Prozess zur Herstellung von chemischen Grundstoffen wie beispielsweise Ethen und Propen aus Erdöl dar. Einsatzstoffe sind typischerweise gesättigte Kohlenwasserstoffe wie Ethan, Propan, Butane oder Naphtha. Im Spaltprozess werden die Kohlenwasserstoffe mit Wasserdampf vermischt und in einem Spaltofen auf Temperaturen von typischerweise 800 bis 850°C erhitzt. Die Verweilzeit in den Rohrschlangen des direkt befeuerten Ofens beträgt zwischen 0,1 bis 0,5 Sekunden.

Die Zudosierung von Wasserdampf dient einerseits der Verringerung der Bildung von Koks an den Rohrinnenwänden, aber auch dazu, das Gleichgewicht der thermischen Reaktion auf die Seite der Zielprodukte (u.a. Ethen und Propen) zu verschieben. Die kurze Verweilzeit im Reaktionsbereich und die anschließende schnelle Abkühlung des Spaltgases dienen der Erhöhung der Selektivität, die für das Steamcracking durch das Verhältnis von Methan zu Propen definiert ist.

Die Kombination der unter den Schritten ii) und iii) genannten Trennverfahren mit dem Steamcracken gemäß Schritt iv) ermöglicht eine sehr hohe Nutzung aller flüchtigen Spaltprodukte des Kunststoffabfalls.

Erfindungsgemäß bedeuten die Bezeichnungen "Styrol-haltig" und "Gew.-% Styrol" im Zusammenhang mit Styrolpolymeren stets "einpolymerisiertes" Styrol.

### Erklärung der Zeichnungen

Fig. 1 zeigt eine exemplarische GC-MS-Analyse der Reaktionsprodukte aus Beispiel 1.
Fig. 2 zeigt eine exemplarische GC-Analyse der Reaktionsprodukte aus Beispiel 2.

Die Erfindung wird durch die folgenden Beispiele, Figuren und Ansprüche illustriert.

### Beispiele

Zur Demonstration der Eignung Styrol-haltiger Kunststoffabfälle werden Polymere im Kolben auf 350 °C bis 450 °C erhitzt. Dabei wird unter dieser Temperatur die mittlere Temperatur des Reaktionsgutes im Innenraum des Reaktionsgefäßes während der Reaktionslaufzeit verstanden.

**Tabelle 2: Erfindungs-Beispiele und Vergleichsbeispiele**

| | Bsp. 1 | Bsp. 2 | V1 | V2 | V3 | V4 | Bsp. 3 | Bsp. 4 |
|---|---|---|---|---|---|---|---|---|
| (K) | PS GPPS 158 N | PS 468N (schlagzäh modifiziertes Polystyrol) | PP Homopolymer | PP Copolymer | PE-LD | PE-LLD | Gemisch aus 60% PS GPPS 158 N und 40 % PP Homopolymer | Gemisch aus 60% PS GPPS 158 N und 40% PE-LD |
| Hauptprodukte der thermischen Spaltung | Styrol-Monomer und Styrol-Oligomere | Styrol-Monomer und Styrol-Oligomere | wachsartige Substanzen | wachsartige Substanzen | wachsartige Substanzen | wachsartige Substanzen | Styrol-Monomer und Styrol-Oligomeren | Styrol-Monomer und Styrol-Oligomeren |

| | | | | | | | | |
|---|---|---|---|---|---|---|---|---|
| V = Vergleichsbeispiele | | | | | | | | |

### Beispiel 1:

Die Zersetzung der Polymerproben wird in einer Glasapparatur durchgeführt, bestehend aus Rundkolben mit Heizmantel, Liebigkühler und Kühlfalle. Das zu zersetzende handelsübliche Polystyrol (PS GPPS 158 N, Hersteller: INEOS Styrolution, Frankfurt) wird im Rundkolben vorgelegt, die Einwaage beträgt 100 g. Mit einer Vakuumpumpe wird in der Apparatur ein Unterdruck erzeugt. Der Restdruck in der Apparatur beträgt 45 mbar. Die Starttemperatur der Reaktion beträgt 370 °C, gemessen zwischen Heizmantel und Kolben.

Bei einer Manteltemperatur von 460 °C startet die Kondensat-Bildung. Bei einer Manteltemperatur von 550 °C ist die Reaktion beendet. Die mittlere Reaktionstemperatur liegt bei 460 °C. Die Reaktionsprodukte werden während der gesamten Versuchslaufzeit in zwei Stufen zunächst in einem Kryostaten bei -40 °C und dann in einer mit flüssigem Stickstoff auf -196 °C gekühlten Kühlfalle aufgefangen. Die Ausbeute an Kondensat beträgt 95,4 % bezogen auf die eingesetzte Menge an Polystyrol.

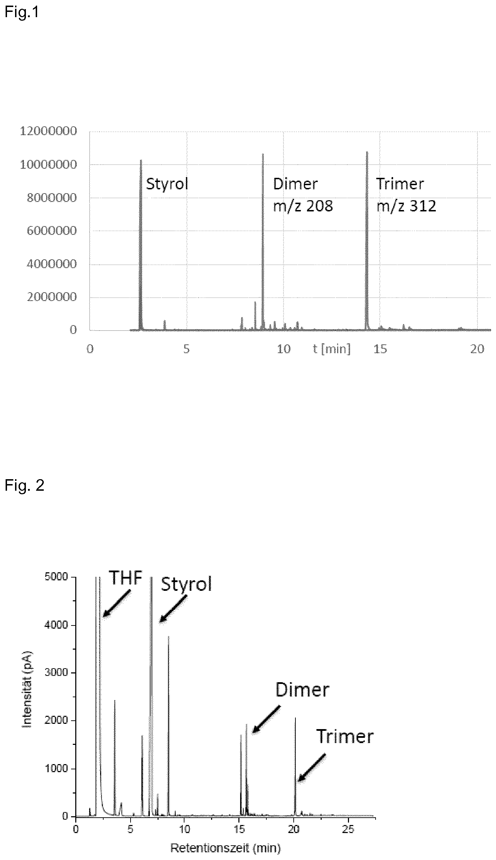

Die entstandenen Reaktionsprodukte werden mittels GC-MS charakterisiert (Agilent 7890A Gaschromatograph, Säule Agilent DB-1 mit He-Trägergas), siehe Fig 1. Die Zusammensetzung der Reaktionsprodukte wird in Tabelle 3 beschrieben.

**Tabelle 3: Zusammensetzung der Reaktionsprodukte aus Beispiel 1**

| Komponente | Anteil im Kondensat | Anteil bezogen auf das Ausgangsmaterial |
|---|---|---|
| Styrol-Monomer | 37,25% | 35,63% |
| Styrol-Dimer | 19,73% | 18,82% |
| Styrol-Trimer | 32,37% | 30,88% |
| Gesamtanteil Styrol und Styrol-Oligomere | 89,35% | 85,23% |

Das Kondensat wird im nächsten Schritt mittels fraktionierender Destillation aufgetrennt. Dazu wird eine Destillation mit einer Vigreux-Kolonne bei durch eine Membranpumpe erzeugtem Unterdruck durchgeführt (Startdruck 50 mbar). Die verschiedenen Fraktionen werden in einer Destillationsspinne aufgefangen, die niedrigsiedende Fraktion wird in einer mit flüssigem Stickstoff gekühlten Kühlfalle aufgefangen. Die Temperatur der Destillation wird dabei von Zimmertemperatur bis 490 °C gesteigert (Manteltemperatur am Kolben). Die erhaltenen Fraktionen werden mittels Gaschromatographie charakterisiert, siehe Tabelle 4. Dabei werden unter "Niedrigsieder" alle flüchtigen Substanzen verstanden, die bei einer niedrigeren Temperatur als das Styrol-Monomer, Styrol-Dimer und Styrol-Trimer aus dem aufzutrennenden Kondensat in die Gasphase übergehen. Unter "Hochsieder" werden alle Substanzen verstanden, die bei höherer Temperatur als das Styrol-Monomer, Styrol-Dimer und Styrol-Trimer aus dem aufzutrennenden Kondensat in die Gasphase übergehen.

**Tabelle 4: Charakterisierung der aufgetrennten Fraktionen aus erfindungsgemäßem Beispiel 1**

| Fraktion | Temperatur Heizmedium (°C) | Anteil Niedrigsieder | Anteil Hochsieder | Anteil Styrol-Monomer | Anteil Styrol-dimer | Anteil Styrol-trimer | Gesamtanteil Styrol und Styrol-Oligomere in der Fraktion | Anteil der Fraktion bezogen auf das Kondensat |
|---|---|---|---|---|---|---|---|---|
| 1 | 82 | 8,94% | - | 91,06% | - | - | 91,06% | 0,80% |
| 2 | 86 | 1,17% | - | 98,83% | - | - | 98,83% | 28,03% |
| 3 | 88 | - | - | 100,00% | - | - | 100,00% | 10,80% |
| 4 | 95 | - | - | 100,00% | - | - | 100,00% | 5,20% |
| 5 | 116 | - | - | 100,00% | - | - | 100,00% | 2,23% |
| 6 | 125 | - | 0,42% | 99,58% | - | - | 99,58% | 1,88% |
| 7 | 133 | - | 0,71% | 99,29% | - | - | 99,29% | 0,48% |
| 8 | 141 | - | 1,56% | 98,44% | - | - | 98,44% | 1,18% |
| 9 | 451 | 4,66% | 21,12% | 39,57% | 31,88 % | 2,76% | 74,21% | 0,50% |
| 10 | 453 | - | 11,71% | 23,07% | 64,20 % | 1,02% | 88,29% | 0,50% |
| 11 | 453 | - | 7,76% | 8,61% | 60,84 % | 22,79% | 92,24% | 2,83% |
| 12 | 490 | - | 8,67% | 8,86% | 79,63 % | 2,83% | 91,06% | 12,83% |
| Kühlfalle | | 0,96% | 4,83% | 86,74% | 6,85% | 0,61% | 91,32% | 0,63% |
| Rückstand im Kolben | | | | | | | | 32,15% |

### Beispiel 2:

Analog zu Beispiel 1 wird der Versuch mit schlagzäh-modifiziertem Polystyrol (PS 486N, Hersteller: INEOS Styrolution) wiederholt. Das Polystyrol PS 486N ist ein schlagzähes amorphes Polystyrol (HIPS) mit einer Schmelz-Volumenfließrate (Melt volume Rate 200 °C/5 kg Belastung, ISO 1133) von etwa 4 cm³/10 min.

Die Starttemperatur der Reaktion beträgt 370 °C, gemessen zwischen Heizmantel und Kolben. Bei einer Manteltemperatur von 450 °C startet die Kondensat-Bildung. Bei einer Manteltemperatur von 550°C ist die Reaktion beendet. Die Reaktionsprodukte werden während der gesamten Versuchslaufzeit in zwei Stufen zunächst in einem Kryostaten bei -40 °C und dann in einer mit flüssigem Stickstoff auf -196 °C gekühlten Kühlfalle aufgefangen. Die Ausbeute an Kondensat beträgt 82,2 % bezogen auf die eingesetzte Menge an Polystyrol. Die entstandenen Reaktionsprodukte werden mittels Gaschromatographie charakterisiert (Agilent 7890A Gaschromatograph, Säule Agilent HP-5 mit Argon-Trägergas, Detektion Flammenionisation, Lösemittel THF), siehe Fig.2.

Die Zusammensetzung der Reaktionsprodukte wird in Tabelle 5 beschrieben.

**Tabelle 5: Zusammensetzung Reaktionsprodukte aus erfindungsgemäßem Beispiel 2**

| Komponente | Anteil im Kondensat | Anteil bezogen auf das Ausgangsmaterial |
|---|---|---|
| Styrol-Monomer | 52,59% | 43,23% |
| Styroldimer | 6,50% | 5,34% |
| Styroltrimer | 10,48% | 8,61% |
| Gesamtanteil Styrol und Styrol-Oligomere | 69,57% | 57,18% |

Das Kondensat wird im nächsten Schritt mittels fraktionierender Destillation aufgetrennt. Dazu wird eine Destillation mit einer Vigreux-Kolonne bei durch eine Membranpumpe erzeugtem Unterdruck durchgeführt. Die verschiedenen Fraktionen werden in einer Destillationsspinne aufgefangen, die niedrigsiedende Fraktion wird in einer mit flüssigem Stickstoff gekühlten Kühlfalle aufgefangen. Die Temperatur der Destillation wird dabei von Zimmertemperatur bis 160 °C gesteigert (Manteltemperatur am Kolben). Die erhaltenen Fraktionen werden mittels Gaschromatographie charakterisiert, siehe Tabelle 6. Dabei werden unter "Niedrigsieder" alle flüchtigen Substanzen verstanden, die bei einer niedrigeren Temperatur als das Styrol-Monomer, Styrol-Dimer und Styrol-Trimer aus dem aufzutrennenden Kondensat in die Gasphase übergehen. Unter "Hochsieder" werden alle Substanzen verstanden, die bei höherer Temperatur als das Styrol-Monomer, Styrol-Dimer und Styrol-Trimer aus dem aufzutrennenden Kondensat in die Gasphase übergehen. Styrol-Monomer, -Dimer und -Trimer sind in der Tabelle 6 in einer Spalte zusammengefasst.

**Tabelle 6: Charakterisierung der aufgetrennten Fraktionen aus Beispiel 2**

| Fraktion | T von Heizmedium (°C) | Anteil Niedrigsieder | Anteil Hochsieder | Anteil Styrol-Monomer | Anteil der Fraktion bezogen auf das Kondensat |
|---|---|---|---|---|---|
| 1 | 78 | 48,88% | 2,34% | 48,78% | 3,35% |
| 2 | 80 | 41,97% | 4,05% | 55,68% | 2,60% |
| 3 | 100 | 15,80% | 7,09% | 77,11 % | 39,55% |
| 4 | 118 | 5,95% | 8,94% | 85,11 % | 5,45% |
| 5 | 130 | 2,42% | 7,73% | 89,85% | 0,45% |
| 6 | 145 | 1,35% | 8,33% | 90,32% | 0,53% |
| 7 | 149 | 0,80% | 15,16 % | 84,04% | 0,13% |
| 8 | 160 | 2,53% | 17,90 % | 79,57% | 0,25% |
| Kühlfalle | | 60,94% | 1,23% | 37,83% | 1,23% |
| Rückstand im Kolben | | | | | 46,48% |

Analoge Experimente werden durchgeführt mit einem Polymerblend aus 85 Gew.-% Polystyrol (A), 8 Gew.-% aromatischem Polycarbonat (B2), 2 Gew.-% üblichen Additiven (B3) und 5 % Fremdstoffen (C).

## Patentansprüche

1. Verfahren zur pyrolytischen Depolymerisation eines Styrol-haltigen KunststoffAbfalls (K), bestehend aus den Komponenten A, B1, B2, B3 und C:
A: 10 bis 100 Gew.-%, bezogen auf die Summe der Komponenten A, B1, B2 und B3, mindestens eines Styrol-haltigen Polymers, enthaltend mindestens 40% Styrol, bevorzugt mindestens 50 Gew.-% Styrol, besonders bevorzugt mindestens 80 Gew.-% Styrol, insbesondere mindestens 85 Gew.-% Styrol, jeweils bezogen auf die Komponente A, und bis zu 60 Gew.-%, bezogen auf die Komponente A, Kautschuk und/oder weitere Comonomere, die den Pyrolyseprozess nicht unterbrechen, insbesondere Acrylnitril, Vinychlorid, Methylmethacrylat und/oder alpha-Methylstyrol;
B1: 0 bis 60 Gew.-%, bezogen auf die Summe aus A, B1, B2 und B3, Polyolefin oder Polyolefin-Mischungen, wie Polyethylen oder Polypropylen;
B2: 0 bis 60 Gew.-%, bezogen auf die Summe aus A, B1, B2 und B3, weitere von A und B1 verschiedene Polymere, wie Polycarbonate, Polyester, Polyamide und/oder Polyvinylchlorid;
B3: 0 bis 20 Gew.-%, bezogen auf die Summe aus A, B1, B2 und B3, übliche Kunststoff-Additive und -Hilfsstoffe;
C: 0 bis 50 Gew.-%, bezogen auf die Summe aus A, B1, B2 und B3, sonstige Fremdstoffe, Schmutz und Feuchtigkeit;
umfassend die folgenden Schritte
i) Zersetzen des Styrol-haltigen Kunststoffabfalls (K) in einem geeigneten Reaktor ohne Katalysator durch Einbringen von thermischer Energie und gegebenenfalls Scherenergie, wobei der zu zersetzende Kunststoffabfall (K) einer Pyrolysezone des Reaktors zugeführt und dort bei einer Temperatur von 250 °C bis 500 °C (gemessene mittlere Materialtemperatur des Reaktionsgutes an der inneren Oberfläche einer Reaktorwand des Reaktors während der Reaktionslaufzeit) pyrolysiert wird, wobei die Verweilzeit des zu pyrolysierenden Kunststoffabfalls (K) in der Pyrolysezone 1 bis 45 Minuten beträgt, und wobei die Styrolkomponente des Styrol-haltigen Kunststoffabfalls (K) zumindest teilweise zu Styrol-Monomeren und Styrol-Oligomeren zersetzt wird;
ii) Abführen und Auffangen der in Schritt i) entstehenden Gase und Kondensation der niedermolekularen Produkte, enthaltend die gebildeten Styrol-Monomere, in einem geeigneten Abscheider;
iii) Auftrennung der kondensierten niedermolekularen Bestandteile des vorherigen Schrittes, enthaltend die gebildeten Styrol-Monomere, mittels fraktionierender Destillation.

2. Verfahren gemäß Anspruch 1, **dadurch gekennzeichnet, dass** in einem weiteren Schritt iv) die in Schritt i) gebildeten sowie gegebenenfalls bereits vorhandenen Styrol-Oligomere zu einem Steamcracker zugeführt werden.

3. Verfahren gemäß Anspruch 1 oder 2, wobei die Komponente A 0 bis 10 Gew.-%, bezogen auf die Komponente A, an einem oder mehreren Comonomeren aus der Gruppe bestehend aus Acrylnitril, Vinylchlorid, Methylmethacrylat und alpha-Methylstyrol enthält.

4. Verfahren gemäß einem der Ansprüche 1 bis 3, wobei der Anteil der Komponente B1 0,1 bis 50 Gew.-%, bezogen auf die Summe der Komponenten A, B1, B2 und B3, beträgt.

5. Verfahren gemäß einem der Ansprüche 1 bis 4, wobei der Anteil der Komponente C 0,1 bis 30 Gew.-%, bevorzugt 0,1 bis 20 Gew.-%, bezogen auf die Summe der Komponenten A, B1, B2 und B3, beträgt.

6. Verfahren gemäß einem der Ansprüche 1 bis 5, wobei Komponente A in einer Menge von mindestens 50 Gew.-%, bezogen auf die Summe der Komponenten A, B1, B2 und B3, vorhanden ist, wobei Komponente B1 in einer Menge von mindestens 10 Gew.-%, bezogen auf die Summe der Komponenten A, B1, B2 und B3, vorhanden ist, und der Anteil der Komponente C 0,1 bis 20 Gew.-%, bezogen auf die Summe der Komponenten A, B1, B2 und B3, liegt.

7. Verfahren gemäß einem der Ansprüche 1 bis 6, wobei in Schritt i) die Pyrolysetemperatur im Bereich von 280 bis 470°C, bevorzugt von 300 bis 450°C, liegt und die Verweilzeit im Bereich von2 bis 30 Minuten, liegt.

8. Verfahren gemäß einem der Ansprüche 1 bis 7, wobei der Pyrolysereaktor in Schritt i) gewählt ist aus der Gruppe bestehend aus Doppelschneckenextrudern, Wirbelschichtreaktoren und Mikrowellenreaktoren.

9. Verfahren gemäß einem der Ansprüche 1 bis 8, wobei in Schritt i) zusätzlich Scherenergie in den Styrol-haltigen Kunststoffabfall (K) eingebracht wird.

10. Verfahren gemäß einem der Ansprüche 1 bis 9, wobei in einem vorgelagerten Schritt o) der Styrol-haltige Kunststoffabfall (K) einer Vorbehandlung unterzogen wird, umfassend einen oder mehrere der folgenden Schritte, wobei die Reihenfolge der Schritte nicht festgelegt ist und Schritte auch mehrfach wiederholt werden können: manuelle Störstoffsortierung, Wäsche, Zerkleinerung, automatische Sortierung in geeigneten Anlagen.

## Claims

1. A process for the pyrolytic depolymerization of a styrene-containing plastics waste (K) consisting of the components A, B1, B2, B3 and C:
A: 10 to 100% by weight, based on the entirety of components A, B1, B2 and B3, of at least one styrene-containing polymer comprising at least 40% of styrene, preferably at least 50% by weight of styrene, particularly preferably at least 80% by weight of styrene, in particular at least 85% by weight of styrene, based in each case on component A, and up to 60% by weight, based on component A, of rubber and/or other comonomers which do not interrupt the pyrolysis process, in particular acrylonitrile, vinyl chloride, methyl methacrylate and/or alphamethylstyrene;
B1: 0 to 60% by weight, based on the entirety of A, B1, B2 and B3, of polyolefin or polyolefin mixtures, such as polyethylene or polypropylene;
B2: 0 to 60% by weight, based on the entirety of A, B1, B2 and B3, of other polymers, differing from A and B1, for example polycarbonates, polyesters, polyamides and/or polyvinyl chloride;
B3: 0 to 20% by weight, based on the entirety of A, B1, B2 and B3, of conventional plastics additives and conventional plastics auxiliaries;
C: 0 to 50% by weight, based on the entirety of A, B1, B2 and B3, of other foreign substances, dirt and moisture;
comprising the following steps:
i) decomposing the styrene-containing plastics waste (K) in a suitable reactor without a catalyst via introduction of thermal energy and optionally of shear energy, where the plastics waste (K) to be decomposed is introduced into a pyrolysis zone of the reactor and is pyrolyzed there at a temperature of 250°C to 500°C (average material temperature of the reaction mixture measured at the inner surface of a reactor wall during the reaction time), where the residence time in the pyrolysis zone of the plastics waste (K) to be pyrolyzed is 1 to 45 minutes, and where the styrene component of the styrene-containing plastics waste (K) is at least partially decomposed to give styrene monomers and styrene oligomers;
ii) discharging and collecting the gases produced in step i) and condensation of the low-molecular-weight products comprising the resultant styrene monomers, in a suitable separator;
iii) separating, by means of fractional distillation, of the condensed low-molecular-weight constituents of the previous step comprising the resultant styrene monomers.

2. The process according to claim 1, **characterized in that** in a further step iv) the styrene oligomers formed in step i), and also any styrene oligomers already present, are introduced into a steam cracker.

3. The process according to claim 1 or 2, where component A comprises 0 to 10% by weight, based on component A, of one or more comonomers from the group consisting of acrylonitrile, vinyl chloride, methyl methacrylate and alphamethylstyrene.

4. The process according to any of claims 1 to 3, where the proportion of component B1 is 0.1 to 50% by weight, based on the entirety of components A, B1, B2 and B3.

5. The process according to any of claims 1 to 4, where the proportion of component C is 0.1 to 30% by weight, preferably 0.1 to 20% by weight, based on the entirety of components A, B1, B2 and B3.

6. The process according to any of claims 1 to 5, where component A is present in a quantity of at least 50% by weight, based on the entirety of components A, B1, B2 and B3, where the component B1 is present in a quantity of at least 10% by weight, based on the entirety of components A, B1, B2 and B3, and the proportion of component C is 0.1 to 20% by weight, based on the entirety of components A, B1, B2 and B3.

7. The process according to any of claims 1 to 6, where the pyrolysis temperature in step i) is in the range of from 280 to 470°C, preferably from 300 to 450°C, and the residence time in step i) is in the range of from 2 to 30 minutes.

8. The process according to any of claims 1 to 7, where the pyrolysis reactor in step i) is selected from the group consisting of twin-screw extruders, fluidized-bed reactors and microwave reactors.

9. The process according to any of claims 1 to 8, where shear energy is additionally introduced in step i) into the styrene-containing plastics waste (K).

10. The process according to any of claims 1 to 9, where in a preceding step o), the styrene-containing plastics waste (K) is subjected to a pretreatment comprising one or more of the following steps, where the sequence of the steps is not fixed and multiple repetition of steps is also possible: manual sorting of impurities, washing, comminution, automatic sorting in suitable systems.

## Revendications

1. Procédé de dépolymérisation pyrolytique d'un déchet plastique (K) contenant du styrène, constitué des composants A, B1, B2, B3 et C :
A : 10 à 100 % en poids, par rapport à la somme des composants A, B1, B2 et B3, d'au moins un polymère contenant du styrène, contenant au moins 40 % de styrène, de préférence au moins 50 % en poids de styrène, d'une manière particulièrement préférée au moins 80 % en poids de styrène, en particulier au moins 85 % en poids de styrène, dans chaque cas par rapport au composant A, et jusqu'à 60 % en poids, par rapport au composant A, de caoutchouc et/ou d'autres comonomères, qui n'interrompent pas le processus de pyrolyse, en particulier l'acrylonitrile, le chlorure de vinyle, le méthacrylate de méthyle et/ou l'alpha-méthylstyrène ;
B1 : 0 à 60 % en poids, par rapport à la somme de A, B1, B2 et B3, d'une polyoléfine ou de mélanges de polyoléfines, telles que le polyéthylène ou le polypropylène ;
B2 : 0 à 60 % en poids, par rapport à la somme de A, B1, B2 et B3, d'autres polymères différents de A et B1, tels que des polycarbonates, des polyesters, des polyamides et/ou du poly(chlorure de vinyle) ;
B3 : 0 à 20 % en poids, par rapport à la somme de A, B1, B2 et B3, d'additifs et adjuvants usuels pour plastiques ;
C : 0 à 50 % en poids, par rapport à la somme de A, B1, B2 et B3, d'autres substances étrangères, de salissure et d'humidité ;
comprenant les étapes suivantes :
i) décomposition du déchet plastique (K) contenant du styrène dans un réacteur approprié sans catalyseur par apport d'énergie thermique et éventuellement d'énergie de cisaillement, le déchet plastique (K) à décomposer étant envoyé dans une zone de pyrolyse du réacteur et y subissant une pyrolyse à une température de 250 °C à 500 °C (température moyenne mesurée du produit de réaction sur la surface interne d'une paroi du réacteur pendant la durée de la réaction), le temps de séjour du déchet plastique (K) à pyrolyser dans la zone de pyrolyse étant de 1 à 45 minutes, et le composant styrène du déchet plastique (K) contenant du styrène étant au moins partiellement décomposé en monomères de styrène et oligomères de styrène ;
ii) évacuation et captage des gaz formés dans l'étape i) et condensation, dans un collecteur approprié, des produits à faible masse moléculaire, contenant les monomères de styrène formés ;
iii) séparation, par distillation fractionnée, des constituants condensés à faible masse moléculaire de l'étape précédente, contenant les monomères de styrène formés.

2. Procédé selon la revendication 1, **caractérisé en ce que**, dans une autre étape iv), on envoie à un vapocraqueur les oligomères de styrène formés dans l'étape i), éventuellement déjà présents.

3. Procédé selon la revendication 1 ou 2, dans lequel le composant A contient 0 à 10 % en poids, par rapport au composant A, d'un ou plusieurs comonomères du groupe consistant en l'acrylonitrile, le chlorure de vinyle, le méthacrylate de méthyle et l'alpha-méthylstyrène.

4. Procédé selon l'une des revendications 1 à 3, dans lequel la proportion du composant B1 est de 0,1 à 50 % en poids par rapport à la somme des composants A, B1, B2 et B3.

5. Procédé selon l'une des revendications 1 à 4, dans lequel la proportion du composant C est de 0,1 à 30 % en poids, de préférence de 0,1 à 20 % en poids, par rapport à la somme des composants A, B1, B2 et B3.

6. Procédé selon l'une des revendications 1 à 5, dans lequel le composant A est présent en une quantité d'au moins 50 % en poids par rapport à la somme des composants A, B1, B2 et B3, le composant B1 étant présent en une quantité d'au moins 10 % en poids par rapport à la somme des composants A, B1, B2 et B3, et la proportion du composant C étant de 0,1 à 20 % en poids par rapport à la somme des composants A, B1, B2 et B3.

7. Procédé selon l'une des revendications 1 à 6, dans lequel dans l'étape i) la température de pyrolyse est comprise dans la plage de 280 à 470 °C, de préférence de 300 à 450 °C, et le temps de séjour est compris dans la plage de 2 à 30 minutes.

8. Procédé selon l'une des revendications 1 à 7, dans lequel le réacteur de pyrolyse est choisi dans l'étape i) dans le groupe consistant en les extrudeuses à double vis, les réacteurs à lit fluidisé et les réacteurs à microondes.

9. Procédé selon l'une des revendications 1 à 8, dans lequel dans l'étape i), on amène en outre de l'énergie de cisaillement dans le déchet plastique (K) contenant du styrène.

10. Procédé selon l'une des revendications 1 à 9, dans lequel, dans une étape préalable o), on soumet le déchet plastique (K) contenant du styrène à un prétraitement comprenant une ou plusieurs des étapes suivantes, la séquence des étapes n'étant pas fixée, et les étapes pouvant aussi être plusieurs fois répétées : tri manuel des impuretés, lavage, broyage, tri automatique dans des installations appropriées.
